# EUROPEAN PATENT SPECIFICATION

(11) **EP 1 471 898 B1**
(45) Date of publication and mention of the grant of the patent: **02.01.2008**
(21) Application number: 03709693.0
(22) Date of filing: 23.01.2003
(51) Int. Cl.: A61K 31/07, A61P 27/02

(54) **LUTEIN/ZEAXANTHIN FOR GLARE PROTECTION**
LUTEIN/ZEAXANTHIN GEGEN BLENDUNG
LUTEINE/ZEAXANTHINE SERVANT A LA PROTECTION CONTRE L'EBLOUISSEMENT

(30) Priority: 30.01.2002 EP 02001909
(43) Date of publication of application: 03.11.2004
(73) Proprietor: DSM IP Assets B.V., 6411 TE Heerlen (NL)
(72) Inventor: BARKER, Felix, Wyncote, PA 19095 (US); GORALCZYK, Regina, D-79639 Grenzach-Wyhlen (DE); SCHALCH, Wolfgang, CH-4103 Bottmingen (CH)
(74) Representative: Schwander, Kuno
(86) International application number: PCT/EP2003/000656
(87) International publication number: WO 2003/063848

(56) References cited:
- GB-A- 2 301 775
- HEINERMANN P.H.: "YELLOW INTROCULAR FILTERS IN FISHES" EXPERIMENTAL BIOLOGY, vol. 43, no. 2, 1984, pages 127-147, XP002242363 GERMANY
- RICHER S.: "armd-pilot (case series) enviromental intervention data" JOURNAL OF THE AMERICAN OPTOMETRIC ASSOCIATION, vol. 70, no. 1, January 1999 (1999-01), pages 24-36, XP009011053 usa
- HAMMOND, B.R. ET AL.: "DENSITY OF THE HUMAN CRYSTALLINE LENS IS RELATED TO THE MACULAR PIGMENT CAROTENOIDS, LUTEIN AND ZEAXANTHIN" OPTOMETRY AND VISION SCIENCE, vol. 74, no. 7, 1997, pages 499-504, XP009011411 USA
- OLMEDILLA, B. ET AL.: "LUTEIN IN PATIENTS WITH CATARACT AND ADE-RELATED MACULAR DEGENERATION: A LONG-TERM SUPPLEMENTATION STUDY" JOURNAL OF THE SCIENCE OF FOOD AND AGRICULTURE, vol. 81, no. 9, July 2001 (2001-07), pages 904-909, XP002242364

## Description

Journal of the Science of Food and Agriculture 2001, 81(9), 904-909 discloses the results of a long-term supplementation study, where patients diagnosed with cataracts or age-related macular degeneration agreed to take three lutein capsules per week. The ophthalmological evaluation showed an average increment in visual acuity of 0.4, and that the glare sensitivity was also improved. Thus, the study organizer conclude that a lutein supplementation at achievable dietary levels increased and maintained serum lutein levels, which were associated with an improvement in the visual function of the patients.

Journal of the American Optometric Association 1999, 70(1), 24-36 deals also with age-related macular degeneration. Beside dark-green, leafy vegetables patients either got additionally spinach or a lutein-based antioxidant.

According to GB 2 301 775 the carotenoids lutein and zeaxanthin are used either separately or in combination to treat age-related macular degeneration. The carotenoids are administered in the form of a pharmaceutical preparation, e.g. a capsule or alternatively as a food e.g. a genetically engineered tomato producing enhanced levels of carotenoid. High dosages of lutein and zeaxanthin are needed to ensure high serum levels necessary for take up of the carotenoids by the macula.

The present invention relates to the reduction of glare or promotion recovery from glare. More particularly, the present invention relates to the reduction of glare or promotion recovery from glare in the darkness, e.g., when steering a vehicle or aircraft under low or dim light conditions such as at night or dawn, or when steering a vehicle in tunnels.

It has been found that administration of a colorant that is capable of being incorporated into eye tissue and/or causing yellowing of eye tissue results in reduction of glare or promotion recovery from glare. A common feature of such colorants is that they when deposited in the eye tissue, particularly the retina, provide a yellow filter that absorbs blue light. Blue light is supposed to be potentially damaging to the retina. Colorants that can be used are lutein or zeaxanthin or esters thereof, or mixtures of the foregoing. Administration of lutein and zeaxanthin has been found to lower the risk for developing age-related macular disease (AMD). However, these compounds are useful to reduce glare or promote recovery from glare also in the absence of AMD. Accordingly, in one aspect the present invention relates to the use of colorant that is capable of being incorporated into eye tissue and/or causing yellowing of eye tissue, selected from lutein, zeaxanthin, or esters thereof, or mixtures of the foregoing, in the manufacture of a composition for reduce glare or promote recovery from glare in the darkness.

In a further embodiment, the present invention relates to the use of a the aforementioned colorants in combination with an anti-oxidant selected from vitamin E or zinc.

Esters of lutein or zeaxanthin are preferably esters of saturated alkanoic acids such as acetic, propionic, palmitic, stearic and succinic acid, mono-unsaturated fatty acids such as oleic acid, and poly-unsaturated fatty acids such as linolic, linoleic, docosahexaenoic and arachidonic acid.

Examples of zinc salts are zinc salts of mineral acids such as zinc sulfate, or of organic acids such as zinc orotate.

The term "vitamin E" refers to natural or racemic α-tocopherol as well as esters thereof such as the acetate. The term "vitamin C" comprises ascorbic acid and esters and salts thereof such as ascorbyl palmitate and sodium ascorbyl phosphate.

Of particular interest is recovery from and the reduction of the physiological effects of glare, especially glare caused by blue light, e.g., when driving in the darkness, i.e. at night or dawn, or in tunnels.

The term "eye tissue" comprises retina, lens, vitreous, retinal pigment epithelium, iris and ciliary body.

The term "composition as used herein denotes any composition that is suitable for administration to the human body, such as pharmaceutical preparations, food or beverage.

A pharmaceutical preparation in accordance with the present invention for reducing glare as promoting recovery from glare may be in any form that is conventional for oral administration, e.g. in solid form such as tablets including effervescent tablets, or soft or hard shell capsules, or in liquid form, such as solutions or suspensions, preferably oily suspension. Besides the active ingredients the pharmaceutical preparation may contain conventional pharmaceutical carrier material, additives and adjuvants, which include water, gelatin, vegetable gums, sugars, vegetable oils,polyalkylene glycols, flavoring agents, preservatives, stabilizers, emulsifying agents, buffers and the like. The medicaments may be in the form of controlled (delayed) release formulations. For the purpose of the invention the colorants as well as optional ingredients as defined earlier hereinabove may be incorporated in food or beverages, such as bakery items, e.g., cake and cookies, lemonades and fruit juices.

In a preferred aspect, the invention relates to the use of colorants as defined earlier hereinabove in the manufacture of a medicament, a food or beverage for reducing glare or promoting recovery from glare in driving at night. Preferably, a combination of lutein and zeaxanthin is used. In such combination these compounds are preferably used in a ratio of 0.1-1.0 : 1.0- 0.1 parts by weight.

In solid pharmaceutical preparations, the compounds selected from lutein, zeaxanthin, or esters thereof, are suitably present in an amount from about 0.1 mg to about 500 mg, preferably from about 1 mg to about 100 mg per dosage unit. In liquid formulations, the aforesaid ingredients are suitably present in an amount of from about 0.1 to about 5 percent by weight based upon the total weight of the composition. If vitamin E is present, its amount is suitably from about 10 to about 1000 mg per dosage unit in solid formulations and from about 0.1 to about 500 mg in liquid formulations. In liquid formulations vitamin E may serve as a carrier for other lipophilic components of the formulations in accordance with the invention and may comprise 99,9 - 50% percent by weight based upon the total weight of the composition. Zinc may be present in an amount of 1 to 100 mg (based on elementary zinc) per dosage unit.

Preferred solid pharmaceutical preparations comprise, per dosage unit, about 6 mg to about 12 mg of lutein, zeaxanthin, or esters thereof, or mixtures of the foregoing; about 200 mg of vitamin E; and 1 mg to about 10 mg of zinc, and, optionally, about 1000 International Units of vitamin A and further optionally, about I mg to about 10 mg of β-carotene. Thus, in a further aspect, the present invention also relates to such preferred solid pharmaceutical preparations.

A suitable daily dosage of the ingredients, lutein, zeaxanthin, or esters thereof, in a pharmaceutical preparation prepared in accordance with the present invention or contained in any food or beverage is, e.g., within the range of from 0.001 mg per kg body weight to about 20 mg per kg body weight. More preferred is a daily dosage of about 0.01 to about 10 mg per kg body weight, and especially preferred is about 0.1 to 1.0 mg per kg body weight per day, based upon the total weight of these components in their unesterified form.

The invention is illustrated further by the Examples given below:

### Example 1

A soft gelatin capsule may be prepared comprising the following ingredients:

| Ingredient | Amount per Capsule |
|---|---|
| Lutein | 10 mg |
| Lecithin | 50 mg |
| Soy bean oil | 200 mg |

### Example 2

A soft gelatin capsule may be prepared comprising the following ingredients:

| Ingredient | Amount per Capsule |
|---|---|
| Lutein | 10 mg |
| Zeaxanthin | 10 mg |
| Lecithin | 50 mg |
| Soy bean oil | 200 mg |

### Example 3

A soft gelatin capsule may be prepared comprising the following ingredients:

| Ingredient | Amount per Capsule |
|---|---|
| Lutein | 6 mg |
| Zeaxanthin | 6 mg |
| Vitamin E (α-d,l-tocopherol) | 200 mg |
| Vitamin C | 500 mg |
| Lecithin | 50 mg |
| Soy bean oil | 200 mg |

### Example 4

A soft gelatin capsule may be prepared comprising the following ingredients:

| Ingredient | Amount per Capsule |
|---|---|
| Lutein | 12 mg |
| Vitamin E (α-d,l-tocopherol) | 200 mg |
| Vitamin C | 500 mg |
| Lecithin | 50 mg |
| Soy bean oil | 200 mg |

### Example 5

A soft gelatin capsule may be prepared comprising the following ingredients:

| Ingredient | Amount per Capsule |
|---|---|
| Zeaxanthin | 12 mg |
| Vitamin E (α-d,l-tocopherol) | 200 mg |
| Vitamin C | 500 mg |
| Lecithin | 50 mg |
| Soy bean oil | 200 mg |

### Example 6

A soft gelatin capsule may be prepared comprising the following ingredients:

| Ingredient | Amount per Capsule |
|---|---|
| Lutein | 6 mg |
| Zeaxanthin | 6 mg |
| β-Carotene | 6 mg |
| Vitamin E (α-d,l-tocopherol) | 200 mg |
| Vitamin C | 500 mg |
| Zinc (as orotate) | 7.5 mg |
| Lecithin | 50 mg |
| Soy bean oil | 200 mg |

### Example 7

A soft gelatin capsule may be prepared comprising the following ingredients:

| Ingredient | Amount per Capsule |
|---|---|
| Lutein | 6 mg |
| Zeaxanthin | 6 mg |
| Vitamin E (α-d,l-tocopherol) | 200 mg |
| Vitamin C | 500 mg |
| Vitamin A | 1000 Int. Units |
| Zinc (as orotate) | 7.5 mg |
| Lecithin | 50 mg |
| Soy bean oil | 200 mg |

### Example 8

A soft gelatin capsule may be prepared comprising the following ingredients:

| Ingredient | Amount per Capsule |
|---|---|
| Lutein | 6 mg |
| Zeaxanthin | 6 mg |
| β-Carotene | 6 mg |
| Vitamin E (α-d,l-tocopherol) | 200 mg |
| Vitamin C | 500 mg |
| Vitamin A | 1000 Int. Units |
| Zinc (as orotate) | 7.5 mg |
| Lecithin | 50 mg |
| Soy bean oil | 200 mg |

## Claims

1. The use of lutein or zeaxanthin, or ester of lutein or zeaxanthin, or any combination thereof in the manufacture of a composition for reducing glare or promoting recovery from glare.

2. The use according to claim 1 wherein the improvement is in reducing glare or promoting recovery from glare when steering a vehicle or aircraft in the darkness.

3. The use according to claim 1 or 2 of a combination of lutein and zeaxanthin.

4. The use according to any one of claims 1-3 wherein the composition is a pharmaceutical composition.

5. The use according to claim 4 wherein the pharmaceutical composition is for oral application and contains per dosage unit an amount of about 0.1 mg to about 500 mg of lutein or zeaxanthin, or mixtures thereof.

6. The use according to any one of claims 1-5 wherein in the composition additionally contains about 10 mg to about 1000 mg of vitamin E per dosage unit.

7. The use according to any one of claims 1-6 wherein the composition additionally contains about 1 mg to about 100 mg of zinc per dosage unit.

8. The use according to any one of claims 1-3 wherein the composition is a food or beverage.

## Patentansprüche

1. Verwendung von Lutein oder Zeaxanthin oder des Esters von Lutein oder Zeaxanthin oder einer beliebigen Kombination davon bei der Herstellung einer Zusammensetzung zur Reduzierung von Blendung oder zur Förderung der Erholung nach Blendung.

2. Verwendung nach Anspruch 1, wobei die Verbesserung in der Reduzierung von Blendung oder der Förderung der Erholung nach Blendung beim Lenken eines Fahrzeugs oder eines Flugzeugs bei Dunkelheit besteht.

3. Verwendung nach Anspruch 1 oder 2 einer Kombination von Lutein und Zeaxanthin.

4. Verwendung nach einem der Ansprüche 1-3, wobei es sich bei der Zusammensetzung um eine pharmazeutische Zusammensetzung handelt.

5. Verwendung nach Anspruch 4, wobei die pharmazeutische Zusammensetzung oral angewendet wird und pro Dosierungseinheit eine Menge von etwa 0,1 mg bis etwa 500 mg an Lutein oder Zeaxanthin oder deren Mischungen enthält.

6. Verwendung nach einem der Ansprüche 1-5, wobei die Zusammensetzung zusätzlich etwa 10 mg bis etwa 1000 mg Vitamin E pro Dosierungseinheit enthält.

7. Verwendung nach einem der Ansprüche 1-6, wobei die Zusammensetzung zusätzlich etwa 1 mg bis etwa 100 mg Zink pro Dosierungseinheit enthält.

8. Verwendung nach einem der Ansprüche 1-3, wobei es sich bei der Zusammensetzung um ein Nahrungsmittel oder ein Getränk handelt.

## Revendications

1. Utilisation de lutéine ou de zéaxanthine, ou d'un ester de lutéine ou de zéaxanthine, ou toute association de ceux-ci dans la fabrication d'une composition pour réduire l'éblouissement ou favoriser la récupération d'un éblouissement.

2. Utilisation selon la revendication 1, dans laquelle l'amélioration consiste à réduire l'éblouissement ou à favoriser la récupération d'une éblouissement pendant la conduite d'un véhicule ou d'un avion dans l'obscurité.

3. Utilisation selon l'une des revendications 1 et 2 d'une combinaison de lutéine et de zéaxanthine.

4. Composition selon l'une quelconque des revendications 1 à 3, dans laquelle la composition est une composition pharmaceutique.

5. Utilisation selon la revendication 4, dans laquelle la composition pharmaceutique sert dans une application orale et comprend, par unité de dosage, une quantité d'environ 0,1 mg à environ 500 mg de lutéine ou de zéaxanthine, ou des mélanges de celles-ci.

6. Utilisation selon l'une quelconque des revendications 1 à 5, dans laquelle la composition comprend en outre environ 10 mg à environ 1000 mg de vitamine E par unité de dosage.

7. Utilisation selon l'une quelconque des revendications 1 à 6, dans laquelle la composition comprend en outre environ 1 mg à environ 100 mg de zinc par unité de dosage.

8. Composition selon l'une quelconque des revendications 1 à 3, dans laquelle la composition est un aliment ou une boisson.
